# EUROPÄISCHE PATENTSCHRIFT

(11) **EP 1 907 342 B1**
(45) Veröffentlichungstag und Bekanntmachung des Hinweises auf die Patenterteilung: **21.12.2011**
(21) Anmeldenummer: 06763996.3
(22) Anmeldetag: 30.06.2006
(51) Int. Cl.: C07C 7/04, C07C 13/277

(54) **VERFAHREN ZUR GEWINNUNG VON CYCLODODECATRIEN DURCH VERDAMPFUNG**
METHOD FOR OBTAINING CYCLODODECATRIENE BY EVAPORATION
PROCEDE DE PRODUCTION DE CYCLODODECATRIENES PAR VAPORISATION

(30) Priorität: 05.07.2005 DE 102005031316
(43) Veröffentlichungstag der Anmeldung: 09.04.2008
(73) Patentinhaber: BASF SE, 67056 Ludwigshafen (DE)
(72) Erfinder: RUMPF, Bernd, 68766 Hockenheim (DE); LANG, Ortmund, 66909 Quirnbach (DE); HAUNERT, Andrea, 68163 Mannheim (DE); GENGER, Thomas, 67245 Lambsheim (DE); MEIER, Anton, 67134 Birkenheide (DE)
(86) Internationale Anmeldenummer: PCT/EP2006/063746
(87) Internationale Veröffentlichungsnummer: WO 2007/003602

(56) Entgegenhaltungen:
- US-A- 3 365 507

## Beschreibung

Die vorliegende Erfindung betrifft ein Verfahren zur Gewinnung von Cyclododecatrien (CDT) aus einer CDT und Schwersieder wie deaktivierten Katalysator, Salze und Polymere enthaltenden Lösung.

Cyclododecatrien ist ein wertvolles Zwischenprodukt für die Herstellung hochwertiger Kunststoffe wie Nylon 6,6; Nylon 6,12; Polyester oder Polyamide.

Die Herstellung von Cyclododecatrien ist beispielsweise in Ullmann's Encyclopedia of Industrial Chemistry, Fifth Edition, Volume A8, 205-207, beschrieben. Dazu wird Butadien in Gegenwart von Ti, Cr oder Ni enthaltenden Katalysatoren cyclotrimerisiert. Die Ausbeute dieser Reaktion ist typischerweise besser als 80 Gew.-%, als Nebenprodukte werden insbesondere Dimere sowie Oligomere des Butadiens erhalten. Vor einer weiteren Reinigung und Aufarbeitung des Wertproduktes muss der Katalysator deaktiviert werden, dies kann beispielsweise durch Zugabe einer wässrigen Natronlauge erfolgen.

Zur Aufarbeitung des Wertproduktes CDT müssen die Dimere und Oligomere sowie der deaktivierte Katalysator zunächst abgetrennt werden. Dazu wird dieser flüssige Produktstrom, im folgenden auch "Reaktionsaustrag" bezeichnet, in einem geeigneten Verdampfersystem partiell verdampft. Die entstehenden Brüden enthalten das Wertprodukt, das schwersiedende Sumpfprodukt wird beispielsweise thermisch verwertet. In den nachfolgenden Aufarbeitungsschritten wird das Wertprodukt weiter gereinigt.

Aufgrund der thermischen Empfindlichkeit des Wertproduktes CDT muss die Schwersiederabtrennung bei möglichst geringen Verweilzeiten und geringen Temperaturen erfolgen. Bevorzugt setzt man daher Dünnschichtverdampfer oder Kurzwegverdampfer - ggf. in Kombination mit einem vorgeschalteten Fallfilmverdampfer, Zwangsumlaufverdampfer oder Zwangsumlaufentspannungsverdampfer - ein. Ein entsprechendes Apparate- und Verfahrenskonzept (Einsatz eines Dünnschichtverdampfers) ist beispielsweise im US-Patent 3365507 offenbart.

Das Verfahren erweist sich jedoch aufgrund der hierbei eingesetzten Apparate als verfahrenstechnisch aufwändig. Nachteilig bei diesem Apparatekonzept sind ferner die vergleichsweise hohen Investitionskosten für die Kombination Fallfilmverdampfer-Dünnschichtverdampfer und die hohen variablen Kosten für den Betrieb des Dünnschichtverdampfers. Weiterhin ist der Einsatz von Verdampferbauarten wie Fallfilmverdampfern, Zwangsumlauf- und Zwangsumlaufentspannungsverdampfern mit erheblichen verfahrenstechnischen Risiken verbunden, da die im Zulaufstrom enthaltenen, hochsiedenden Komponenten sowie die evtl. bei der Eindampfung entstehenden Zersetzungsprodukte zur Belagbildung an heißen Flächen neigen. Darüber hinaus kann es auch in Dünnschichtverdampfern zur Bildung von Belägen - z. B. am innenliegenden Wischersystem - kommen, was zu Betriebsausfällen führen kann.

Aufgabe der vorliegenden Erfindung war es daher, ein Verfahren zur Verdampfung des Wertproduktes CDT aus dem Reaktionsaustrag zu finden, das CDT unter Vermeidung der o.g. Nachteile mit gleicher oder verbesserter Qualität bezüglich Farbe, Farbstabilität, Geruch und Reinheit liefert. Darüber hinaus waren die Verluste an Wertprodukt durch Restgehalte im Sumpfaustrag zu minimieren.

Demgemäß wurde ein Verfahren zur Gewinnung von Cyclododecatrien (CDT) aus einer CDT und Schwersieder wie deaktivierten Katalysator und Polymere enthaltenden Lösung gefunden, welches dadurch gekennzeichnet ist, dass man die Lösung einem Vorwärmer zuführt und erwärmt, anschließend über eine nachgeschaltete Druckhaltevorrichtung entspannt und das erhaltene, zweiphasige Gemisch einem Wendelrohrverdampfer zuführt und dort durch partielle Verdampfung die flüssige Phase in ihrem Gehalt an CDT verringert und einen gasförmigen Produktstrom mit einer erhöhten Konzentration an CDT ableitet.

Es wurde erstaunlicherweise gefunden, dass man erfindungsgemäß die Schwersiederabtrennung in einem vergleichsweise einfach aufgebauten Apparat - dem Wendelrohrverdampfer (WRV) - ohne externe Durchmischung des Flüssigkeitsfilmes und unter Vermeidung von Belagsbildung an den beheizten Wänden durchführen kann. Das hätte der Fachmann nicht erwartet, da im Vergleich zum herkömmlichen Dünnschichtverdampfer deutlich größere Wärmestromdichten und daraus resultierend deutlich größere treibende Temperaturdifferenzen im Wendelrohrverdampfer vorliegen, was üblicherweise eine Zunahme der Polymerisation und der Belagbildung zur Folge hat.

Wendelrohrverdampfer sind allgemein bekannt, sie sind beispielsweise in der US-Anmeldung 3.550.669, beschrieben. Hier wird ein Verdampfungsapparat beschrieben, bei dem die mechanische Krafteinwirkung zum Freihalten der Wärmetauschfläche nicht durch rotierende Einbauten, sondern durch Strömungskräfte bewirkt wird. Dieser Verdampfungsapparat besteht aus nur einem einzelnen, gewendelten Rohr, das von au-βen beheizt wird. Dieser Einrohrverdampfer wird nun so betrieben, dass die Lösung bzw. Suspension unter Druck überhitzt in den Apparat eingespeist wird, so dass bereits am Anfang des Apparates ein Teil der flüchtigen Bestandteile der Lösung ausdampfen. Dieser Dampf übernimmt die Funktion des Transportes der zäher werdenden Lösung bzw. Suspension durch den Apparat und sorgt für das Freihalten der Wärmeübertragerfläche.

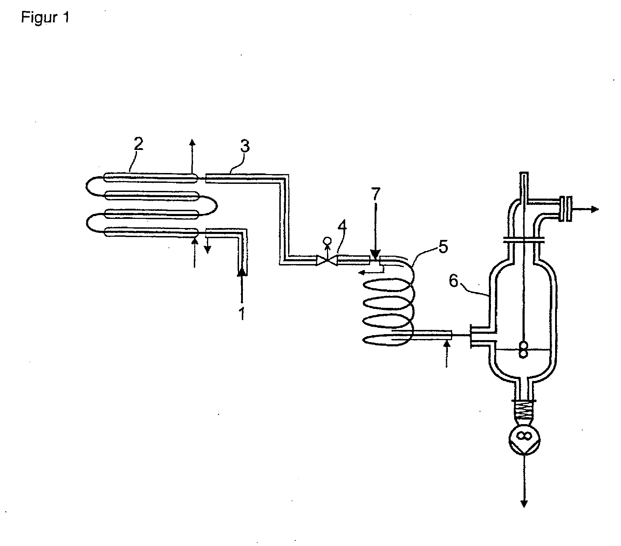

Das erfindungsgemäße Verfahren wird im folgenden anhand der Figur exemplarisch näher beschrieben.

Die aufzuarbeitende Lösung (Reaktionsaustrag) mit einer Konzentration an CDT von typischerweise 20 - 80 Gew.-% (sowie enthaltend Lösungsmittel (Toluol), Schwersieder (Polymere), deaktivierten Katalysator) wird über Leitung (1) einem beispielsweise mit Wärmeträgeröl betriebenen Vorwärmer (2) zugeleitet : und aufgeheizt. Die Vorerwärmung liegt je nach Druck üblicherweise zwischen 80°C bis 250°C. Die erwärmte Lösung wird über Leitung (3) aus dem Vorwärmer abgeführt. Der Druck im Vorwärmer wird durch ein nachgeschaltetes Druckhalteventil (4) so eingestellt, dass an keiner Stelle im Vorwärmer eine Verdampfung der Lösung auftritt. Als Vorwärmer können herkömmliche Apparatetypen wie Rohrbündelapparate, Plattenwärmeübertrager, Spiralwärmeübertrager o.ä. eingesetzt werden. Die erwärmte Lösung wird nach dem Druckhalteventil (4) entspannt und dem von außen beheizten Wendelrohrverdampfer (5) zugeführt.

Die Produkterwärmung im Wedelrohrverdampfer liegt je nach Druck üblicherweise zwischen 100°C bis 270°C.

Durch entsprechende Wahl der Geometrie, des Gesamtmengenstromes und des Gasanteiles nach der Entspannung wird im Rohr eine wellige Filmströmung eingestellt. Damit wird ein intensiver Wärme- und Stoffaustausch geschaffen. Durch die hohen Durchsätze liegen hohe Wandschubspannungen vor, sodass der Aufbau von Anbackungen an den beheizten Wänden wirkungsvoll vermieden wird.

Die zu erreichende Abdampfrate - und damit die Konzentration des Wertproduktes im Sumpfprodukt - wird durch die Wahl der Beheizungstemperatur und des Druckes im nachgeschalteten Brüdenabscheider festgelegt und kann beispielsweise durch Versuche ermittelt werden. Unter Abdampfrate versteht man hierbei das Verhältnis aus Destillatmenge und Zulaufmenge.

Die Beheizung des Wendelrohrverdampfers kann z. B. durch kondensierenden Dampf oder mit Hilfe eines temperierten Ölkreislaufes erfolgen. Auch eine elektrische Beheizung ist möglich.

Durch Zugabe von Fremddampf oder Inertgas mittels Leitung (7) kann der Partialdruck der verdampfbaren Komponente abgesenkt und die Gasgeschwindigkeit erhöht werden.

Dies kann sich empfehlen, um die gewünschte Strömungsform im Wendelrohrverdampfer zu erreichen bzw. um die Verweilzeit der Lösung im Wendelrohrverdampfer einzustellen oder durch Strippen Leichtsiederreste aus der Lösung zu entfernen. Die Strippgasmenge zum Wendelrohrverdampfer liegt bei 0 bis 50 Gew.-% bezogen auf den Gesamfizulaufstrom (Produkt + Strippgas), bevorzugt werden 0 bis 20 Gew.-% zugeführt.

In einem nachgeschalteten Brüdenabscheider (6) werden Flüssigkeit und Gas voneinander getrennt. Der Brüdenstrom kann in herkömmlichen Kondensatoren z. B. Rohrbündelapparaten oder Quenchkondensatoren - kondensiert werden. Die anfallenden Kondensate, die im wesentlichen das Wertprodukt CDT enthalten, können in herkömmlichen Destillationseinrichtungen aufgearbeitet oder direkt weiter verwertet werden. Üblicherweise liegt die Konzentration an CDT im Kondensat zwischen 40 bis 90 Gew.-%.

Der Sumpfstrom aus dem Brüdenabscheider enthält im wesentlichen die bei der Reaktion gebildeten Schwersieder sowie deaktivierten Katalysator, der Gehalt an Wertprodukt liegt hier je nach Fahrweise bei weniger als 20 Gew. -% an CDT, bevorzugt weniger als 5 Gew. - %, besonders bevorzugt weniger als 1 Gew. - % CDT, es können sogar Restanteile an CDT kleiner als 0.5 Gew.-% erreicht werden.

Der Druck im Brüdenraum wird auf 1 bis 10⁴ mbar, bevorzugt 1 bis 10³ mbar, besonders bevorzugt 1 bis 200 mbar eingestellt. Im Fall erhöhter Anforderungen kann sich hier ein Druck von 1 bis 100 mbar besonders empfehlen.

Generell kann die Verweilzeit durch die Strömungsgeschwindigkeit bzw. die Geometrie des Wendelrohrverdampfers (Durchmesser und Länge) vorgegeben werden. Die Verweilzeit im Wendelrohrverdampfer und dem zugehörigen Rohrleitungssystem wird vorteilhafterweise auf 0,5 bis 10 Minuten, bevorzugt 0,5 bis 2 Minuten, begrenzt. Dadurch wird eine mögliche weitere Polymerisation und die thermische Zersetzung des Wertproduktes vermindert.

Das Verfahren wird in der Regel kontinuierlich durchgeführt, prinzipiell ist es aber auch möglich, die Abtrennung absatzweise durchzuführen.

Ebenso ist es möglich, mehrere Wendelrohrverdampfer zu einer Verdampferkaskade hintereinander zu schalten und den zulaufenden Produktstrom mehrstufig zu verdampfen. In dieser Variante kann es vorteilhaft sein, die Verdampferkaskade bei unterschiedlichen Drücken zu betreiben. Gegebenfalls kann die Verdampferstufe auch teilweise Wärmeintegriert betrieben werden. Die Wahl der unterschiedlichen Druckniveaus der Verdampferstufen kann durch den Fachmann durch Rechnungen und/oder Versuche ermittelt werden.

Es ist auch möglich, einen Teil des dem Wendelrohrverdampfers entnommenen, flüssigen Stromes erneut in den Wendelrohrverdampfer zur weiteren Eindampfung zurückzuführen. Hierdurch kann die Aufreinigung weiter verbessert werden.

Des weiteren kann es sich empfehlen, das Wendelrohr innen und / oder außen zu berippen. Darunter versteht man die Anbringung von Rippen an der Innen- oder Außenseite des Wendelrohres, dies hat den Vorteil, dass dadurch die Leistung des Wendelrohres verbessert wird. Diese Verbesserung kommt sowohl durch Bereitstellung einer größeren Wärmeübertragungsfläche als auch durch die Erzeugung von zusätzlichen Turbulenzen zustande. Weiterhin kann man das Wendelrohr innen vollständig oder teilweise mit Drahtgestricken ausstatten. Hierunter versteht man das Einbringen von Drahtgestricken in das Wendelrohr, wodurch der Wärme- und Stoffübergang verbessert wird.

Durch die entsprechende Wahl des Betriebspunktes des Wendelrohrverdampfers werden sehr hohe flächenspezifische Leistungen bei geringen Verweilzeiten erzielt. So konnten im Rahmen von Laborversuchen bis zu 8 kg/h einer CDT enthaltenden Lösung problemlos durch ein gewendeltes Rohr mit einem Innendurchmesser von 7 mm durchgesetzt werden.

Aufgrund der geringen Verweilzeit der Lösung bei höheren Temperaturen wird bei dem erfindungsgemäßen Verfahren die Bildung von Polymerisaten infolge zu hoher thermischer Belastung wirksam verhindert, sodass die Verluste an CDT durch Polymerisation im Verdampfersystem entgegen bisheriger Erfahrungen mit herkömmlichen Verdampferkonzepten kleiner als 1 Gew.-% bleiben. Es wird somit eine neue und verfahrenstechnisch einfache Lösung zur Gewinnung von CDT bereitgestellt, welche neben einem geringen apparativen Aufwand hohe Standzeiten und geringe Betriebskosten ermöglicht.

### Beispiele

### Beispiel 1 (nicht erfindungsgemäß):

Aufkonzentration einer Lösung, die 50 Gew.-% CDT enthielt, in einem Fallfilmverdampfer. Die Anlage war mit einem Verdampferrohr 25*2*3000 mm (wärmeübertragende Fläche ca. 0.2 m²) ausgestattet. Der Druck im Brüdenraum wurde auf 200 mbar eingestellt. Die Zulaufmenge betrug 4 kg/h Produkt. Die Zulauftemperatur des eingesetzten Wärmeträgeröles betrug ca. 190°C. Die zu Beginn des Versuches erzielte Abdampfrate betrug ca. 90 %, damit stellte sich ein pauschaler Verlust an Wertprodukt im Sumpf von 1 Gew.-% (bezogen auf die Menge an Wertprodukt im Zulauf) ein. Im Laufe des Versuches musste die Zulauftemperatur des Wärmeträgeröles in mehreren Schritten auf bis zu 220°C angehoben werden, um die Abdampfrate annähernd konstant zu halten. Der Versuch musste nach 3 Tagen Laufzeit abgebrochen werden. Es zeigte sich eine starke und irreversible Belegung des Verdampferrohres.

### Beispiel 2 (erfindungsgemäß):

Aufkonzentration einer Lösung, die 50 Gew.-% CDT enthielt, in einer Wendelrohrverdampferanlage gemäß Figur. Die Anlage war mit einer Glaswendel (1= 6 m, Innendurchmesser 7 mm, wärmeübertragende Fläche ca. 0.19 m²) ausgestattet. Der Druck im Brüdenraum wurde auf 200 mbar eingestellt. Die Zulaufmenge betrug 8 kg/h Produkt. Die Beheizungstemperatur im Vorwärmer betrug 220°C, im Wendefrohr 220°C. Die erzielte Abdampfrate betrug 90. %, damit stellte sich ein pauschaler Verlust an Wertprodukt im Sumpf von 0.8 Gew.-% (bezogen auf die Menge an CDT im Zulauf) ein. Eine irreversible Belegung der Heizflächen konnte auch nach mehreren Tagen Betriebszeit nicht beobachtet werden.

## Patentansprüche

1. Verfahren zur Gewinnung von Cyclododecatrien (CDT) aus einer CDT und Schwersieder wie deaktivierten Katalysator und Polymere enthaltenden Lösung, **dadurch gekennzeichnet, dass** man die Lösung einem Vorwärmer zuführt und erwärmt, anschließend über eine nachgeschaltete Druckhaltevorrichtung entspannt und das erhaltene, zweiphasige Gemisch einem Wendelrohrverdampfer zuführt und dort durch partielle Verdampfung die flüssige Phase in ihrem Gehalt an CDT verringert und einen gasförmigen Produktstrom mit einer erhöhten Konzentration an CDT ableitet.

2. Verfahren nach Anspruch 1, **dadurch gekennzeichnet, dass** man in einem einmaligen Durchgang durch den Wendelrohrverdampfer den Anteil an CDT in der flüssigen Phase auf einen Gehalt kleiner 5 Gew.-% CDT reduziert.

3. Verfahren nach Anspruch 1, **dadurch gekennzeichnet, dass** man in einem einmaligen Durchgang durch den Wendelrohrverdampfer den Anteil an CDT in der flüssigen Phase auf einen Gehalt kleiner 0.5 Gew.-% CDT reduziert.

4. Verfahren nach Ansprüchen 1 bis 3, **dadurch gekennzeichnet, dass** man einen Teil des dem Wendelrohrverdampfers entnommenen, flüssigen Stromes erneut dem Wendelrohrverdampfer zur weiteren Eindampfung zuführt.

5. Verfahren nach Ansprüchen 1 bis 4, **dadurch gekennzeichnet, dass** man in dem Wendelrohrverdampfer Ventile oder Drosseln zur intensiveren Durchmischung der flüssigen Phase einsetzt.

6. Verfahren nach Ansprüchen 1 bis 5, **dadurch gekennzeichnet, dass** man hinter der Entspannungsvorrichtung ein Strippgas zugibt.

7. Verfahren nach Ansprüchen 1 bis 6, **dadurch gekennzeichnet, dass** man im Wendelrohrverdampfer ein innen und / oder außen beripptes Rohr einsetzt.

8. Verfahren nach Ansprüchen 1 bis 7, **dadurch gekennzeichnet, dass** man den Wendelrohrverdampfer innen mit einem Drahtgestrick ausstattet.

9. Verfahren nach Ansprüchen 1 bis 8, **dadurch gekennzeichnet, dass** man den aus dem Wendelrohrverdampfer abgeführten zweiphasigen Strom einem nachgeschalteten Brüdenabscheider zuleitet und den Brüdenabscheider bei einem Druck von 1 bis 400 mbar betreibt.

10. Verfahren nach Ansprüchen 1 bis 8, **dadurch gekennzeichnet, dass** man den aus dem Wendelrohrverdampfer abgeführten zweiphasigen Strom einem nachgeschalteten Brüdenabscheider zuleitet und den Brüdenabscheider bei einem Druck von 1 bis 200 mbar betreibt.

11. Verfahren nach Ansprüchen 1 bis 10, **dadurch gekennzeichnet, dass** man den aus dem Wendelrohrverdampfer abgeführten gasförmigen Strom in einem Kondensator partiell oder vollständig kondensiert.

12. Verfahren gemäß Ansprüchen 1 bis 11, **dadurch gekennzeichnet, dass** man zwei oder mehrere Wendelrohrverdampfer zu einer Verdampferkaskade hintereinander schaltet und das der Verdampferkaskade zulaufende Produkt stufenweise eindampft.

13. Verfahren gemäß Anspruch 12, **dadurch gekennzeichnet, dass** man die Verdampferkaskade bei unterschiedlichen Drücken betreibt.

14. Verfahren gemäß Anspruch 13, **dadurch gekennzeichnet, dass** man die Verdampferkaskade wärmeintegriert betreibt.

## Claims

1. A process for recovering cyclododecatriene (CDT) from a solution comprising CDT and high boilers such as deactivated catalyst and polymers, which comprises feeding the solution into a preheater and heating it, subsequently depressurizing it through a downstream pressure maintenance device and feeding the resulting two-phase mixture into a helical tube evaporator and there reducing the CDT content of the liquid phase by partial evaporation and discharging a gaseous product stream having an increased concentration of CDT.

2. The process according to claim 1, wherein the proportion of CDT in the liquid phase is reduced to a content of less than 5% by weight of CDT in a single pass through the helical tube evaporator.

3. The process according to claim 1, wherein the proportion of CDT in the liquid phase is reduced to a content of less than 0.5% by weight of CDT in a single pass through the helical tube evaporator.

4. The process according to any of claims 1 to 3, wherein part of the liquid stream taken off from the helical tube evaporator is fed back into the helical tube evaporator for further evaporation.

5. The process according to any of claims 1 to 4, wherein valves or restrictors are used in the helical tube evaporator to achieve more intense mixing of the liquid phase.

6. The process according to any of claims 1 to 5, wherein a stripping gas is introduced downstream of the depressurization device.

7. The process according to any of claims 1 to 6, wherein an internally and/or externally ribbed tube is used in the helical tube evaporator.

8. The process according to any of claims 1 to 7, wherein the interior of the helical tube evaporator is provided with a knitted wire mesh.

9. The process according to any of claims 1 to 8, wherein the two-phase stream discharged from the helical tube evaporator is introduced into a downstream vapor separator and the vapor separator is operated at a pressure of from 1 to 400 mbar.

10. The process according to any of claims 1 to 8, wherein the two-phase stream discharged from the helical tube evaporator is introduced into a downstream vapor separator and the vapor separator is operated at a pressure of from 1 to 200 mbar.

11. The process according to any of claims 1 to 10, wherein the gaseous stream discharged from the helical tube evaporator is partially or completely condensed in a condenser.

12. The process according to any of claims 1 to 11, wherein two or more helical tube evaporators are connected in series to form an evaporator cascade and the product flowing into the evaporator cascade is evaporated in stages.

13. The process according to claim 12, wherein the evaporator cascade is operated at different pressures.

14. The process according to claim 13, wherein the evaporator cascade is operated with thermal integration.

## Revendications

1. Procédé d'obtention de cyclododécatriène (CDT) à partir d'une solution contenant du CDT et des composés de point d'ébullition élevé tels qu'un catalyseur désactivé et des polymères, **caractérisé en ce que** la solution est introduite dans un dispositif de préchauffage et chauffée, puis détendue par un dispositif de maintien de la pression connecté en aval, et le mélange biphasé obtenu est introduit dans un évaporateur à tube hélicoïdal et la teneur en CDT de la phase liquide y est réduite par évaporation partielle, et un courant de produits gazeux ayant une concentration élevée en CDT est dérivé.

2. Procédé selon la revendication 1, **caractérisé en ce que** la proportion de CDT dans la phase liquide est réduite à une teneur inférieure à 5 % en poids de CDT par un passage unique dans l'évaporateur à tube hélicoïdal.

3. Procédé selon la revendication 1, **caractérisé en ce que** la proportion de CDT dans la phase liquide est réduite à une teneur inférieure à 0,5 % en poids de CDT par un passage unique dans l'évaporateur à tube hélicoïdal.

4. Procédé selon les revendications 1 à 3, **caractérisé en ce qu'**une partie du courant liquide soutiré de l'évaporateur à tube hélicoïdal est de nouveau introduite dans l'évaporateur à tube hélicoïdal pour une évaporation supplémentaire.

5. Procédé selon les revendications 1 à 4, **caractérisé en ce que** des vannes ou des rétrécissements sont utilisés dans l'évaporateur à tube hélicoïdal pour le mélange intensif de la phase liquide.

6. Procédé selon les revendications 1 à 5, **caractérisé en ce qu'**un gaz d'extraction est ajouté après le dispositif de détente.

7. Procédé selon les revendications 1 à 6, **caractérisé en ce qu'**un tube intérieur et/ou extérieur à ailettes est utilisé dans l'évaporateur à tube hélicoïdal.

8. Procédé selon les revendications 1 à 7, **caractérisé en ce que** l'évaporateur à tube hélicoïdal est muni à l'intérieur d'un tricot métallique.

9. Procédé selon les revendications 1 à 8, **caractérisé en ce que** le courant biphasé déchargé de l'évaporateur à tube hélicoïdal est introduit dans un séparateur de vapeur connecté en aval et le séparateur de vapeur est exploité à une pression de 1 à 400 mbar.

10. Procédé selon les revendications 1 à 8, **caractérisé en ce que** le courant biphasé déchargé de l'évaporateur à tube hélicoïdal est introduit dans un séparateur de vapeur connecté en aval et le séparateur de vapeur est exploité à une pression de 1 à 200 mbar.

11. Procédé selon les revendications 1 à 10, **caractérisé en ce que** le courant gazeux déchargé de l'évaporateur à tube hélicoïdal est condensé en partie ou en totalité dans un condensateur.

12. Procédé selon les revendications 1 à 11, **caractérisé en ce que** deux évaporateurs à tube hélicoïdal ou plus sont connectés en série en une cascade d'évaporateurs et le produit introduit dans la cascade d'évaporateurs est évaporé par étapes.

13. Procédé selon la revendication 12, **caractérisé en ce que** la cascade d'évaporateurs est exploitée à différentes pressions.

14. Procédé selon la revendication 13, **caractérisé en ce que** la cascade d'évaporateurs est exploitée avec intégration thermique.
